# EUROPEAN PATENT APPLICATION

(11) **EP 4 803 511 A1**
(43) Date of publication of application: **09.09.2026**
(21) Application number: 24884886.3
(22) Date of filing: 31.10.2024
(51) Int. Cl.: C07D 327/04, A61K 31/39, A61P 17/00, A61P 17/18

(54) **SMALL MOLECULE COMPOUND HAVING SULFONIC ACID OR SULFINIC ACID LACTONE STRUCTURE, COMPOSITION AND USE THEREOF**

(30) Priority: 01.11.2023 CN 202311449470
(71) Applicant: Hangzhou Phecdamed Co., Ltd., Hangzhou, Zhejiang 311100 (CN)
(72) Inventor: LIU, Dong, Hangzhou, Zhejiang 311100 (CN)
(74) Representative: Viering, Jentschura & Partner mbB Patent- und Rechtsanwälte
(86) International application number: PCT/CN2024/128828
(87) International publication number: WO 2025/092886

(57) **Abstract**

The present application discloses a small molecule compound having sulfonic acid or sulfinic acid lactone structure, composition and use thereof. The present application provides a class of small molecule compounds with a sulfonic acid or a sulfinic acid lactone structure, which have been verified to have extremely low skin irritation, excellent permeability and high stability, and can not only significantly scavenge reactive oxygen species and reduce oxidative stress, but also alleviate skin inflammation, have excellent ability to protect and repair skin cells and can be used as new raw materials for cosmetics.

## Description

### CROSS REFERENCE TO RELATED APPLICATIONS

This patent application claims priority to the Chinese patent application No. 2023114494701 filed Nov. 01, 2023, entitled "Small Molecule Compound Having Sulfonic acid or Sulfinic acid Lactone Structure, Composition and Use Thereof", which is incorporated herein by reference in its entirety.

### FIELD OF THE INVENTION

The present invention relates to the field of biochemistry, and in particular to small molecule compound having sulfonic acid or sulfinic acid lactone structure, composition and use thereof.

### BACKGROUND OF THE INVENTION

Protecting and repairing damaged skin is usually an important function that needs to be provided in skin care products or cosmetics. Factors that cause damage to the skin include hormone levels, excessive sun exposure, haze, and improper diet, and the like. Skin inflammation and damage can easily cause skin roughness, hyperpigmentation, collagen reduction, and reduced skin antioxidant capacity, and can easily accelerate skin aging for a long time. Studies have shown that quenching ROS and scavenging harmful oxygen radicals can alleviate skin inflammation and repair damaged skin. In the existing cosmetics, although traditional antioxidants such as vitamin C, vitamin E, nicotinamide, and arbutin have a certain ROS scavenging capability, there are still serious problems such as severe irritation, weak stability and poor skin permeability.

Therefore, it is of great value to develop new cosmetic raw materials that are high permeable, mildly irritating, capable of quenching ROS, scavenging harmful oxygen radicals, and alleviating skin inflammation.

### SUMMARY OF THE INVENTION

An object of the present invention is to provide a small molecule compound having a sulfonic acid or a sulfinic acid lactone structure.

Another object of the present invention is to provide a composition.

Another object of the present invention is to provide a method for alleviating skin inflammation.

Another object of the present invention is to provide a method for reducing reactive oxygen species in skin cells.

Another object of the present invention is to provide uses of the above compound or composition.

In order to solve the above technical problems, a first aspect of the present invention provides a compound having a structure represented by the following general formula I, or a salt, stereoisomer or solvate thereof, 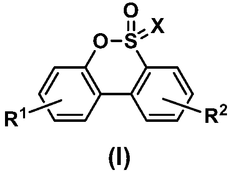 wherein X is oxygen or absent;
R¹ and R² are each independently hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, -OC(O)R¹⁻³, -OCH₂OC(O)R¹⁻⁴, halogen, cyano, nitro, -C(O)R¹⁻⁵, or -C(O)OR¹⁻⁶;
R¹⁻¹, R¹⁻², R¹⁻³, R¹⁻⁴, R¹⁻⁵ and R¹⁻⁶ are each independently hydrogen, C₁₋₆ alkyl, halogen-substituted C₁₋₆ alkyl or -NR^{a}R^{b};
R^{a} and R^{b} are independently hydrogen, C₁₋₆ alkyl or halogen-substituted C₁₋₆ alkyl.

In some preferred embodiments, X is oxygen.

In some preferred embodiments, R¹ and R² are each independently hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, -OC(O)R¹⁻³ or -OCH₂OC(O)R¹⁻⁴.

In some preferred embodiments, R¹ and R² are each independently a hydroxy, a C₁₋₆ alkoxy,

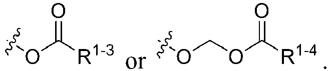

In some preferred embodiments, the C₁₋₆ alkyl is C₁₋₄ alkyl; more preferably, the C₁₋₄ alkyl is methyl, ethyl, n-propyl, isopropyl, n-butyl, isobutyl or tert-butyl.

In some preferred embodiments, the C₁₋₆ alkoxy is a C₁₋₄ alkoxy; more preferably, the C₁₋₄ alkoxy is methoxy, ethoxy, n-propoxy, isopropoxy, n-butoxy, isobutoxy or tert-butoxy.

In some preferred embodiments, the halogen is fluorine, chlorine, bromine or iodine.

In some preferred embodiments, the halogen-substituted C₁₋₆ alkyl is a halogen-substituted C₁₋₄ alkyl; more preferably a halogen-substituted methyl, a halogen-substituted ethyl, a halogen-substituted n-propyl, a halogen-substituted isopropyl, a halogen-substituted n-butyl, a halogen-substituted isobutyl, or a halogen-substituted tert-butyl.

In some preferred embodiments, R¹ is hydroxy, methoxy, ethoxy, n-propoxy, isopropoxy, n-butoxy, isobutoxy, tert-butoxy, 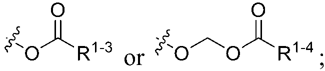 more preferably, R¹ is hydroxy, methoxy,

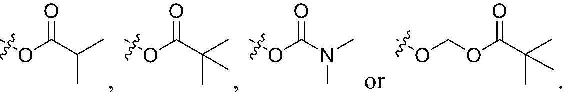

In some preferred embodiments, R² is hydroxy, methoxy, ethoxy, n-propoxy, isopropoxy, n-butoxy, isobutoxy, tert-butoxy, 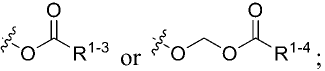 more preferably, R² is hydroxy, methoxy, 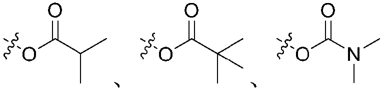 or

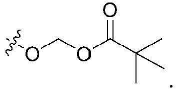

In some preferred embodiments, R¹⁻³ is methyl, ethyl, n-propyl, isopropyl, n-butyl, isobutyl, tert-butyl, or 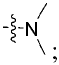 more preferably, R¹⁻³ is isopropyl, tert-butyl or

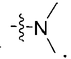

In some preferred embodiments, R¹⁻⁴ is methyl, ethyl, n-propyl, isopropyl, n-butyl, isobutyl, tert-butyl, or 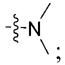 more preferably, R¹⁻⁴ is tert-butyl.

In some preferred embodiments, the compound is selected from any one of the following:

| Name | Structural Formula |
|---|---|
| I-1 | 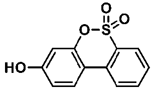 |
| I-2 | 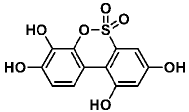 |
| I-3 | 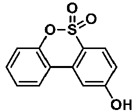 |
| I-4 | 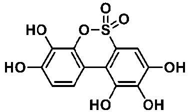 |
| I-5 | 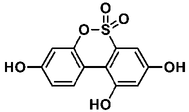 |
| I-6 | 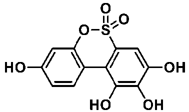 |
| I-7 | 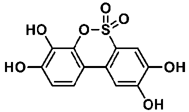 |
| I-8 | 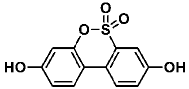 |
| I-9 | 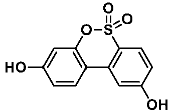 |
| I-10 | 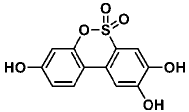 |
| I-11 | 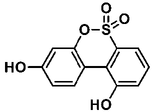 |
| I-12 | 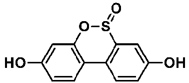 |
| I-13 | 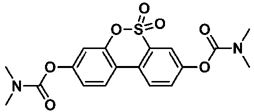 |
| I-14 | 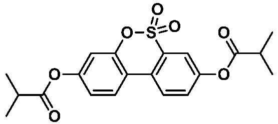 |
| I-15 | 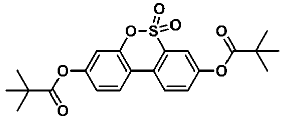 |
| I-16 | 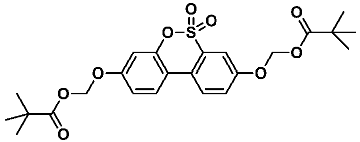 |
| I-17 | 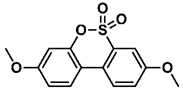 |
| I-18 | 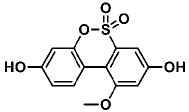 |
| I-19 | 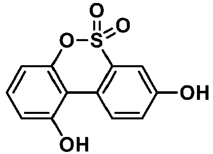 |
| I-20 | 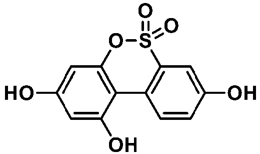 |
| I-21 | 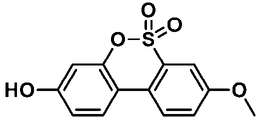 |
| I-22 | 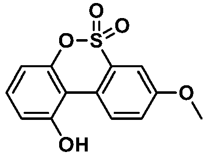 |
| I-23 | 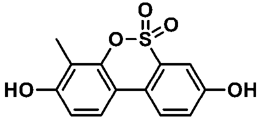 |
| I-24 | 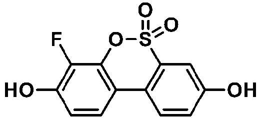 |
| I-25 | 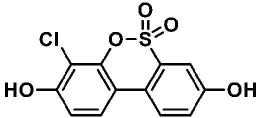 |
| I-26 | 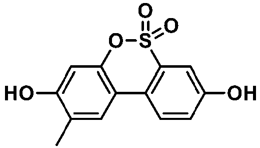 |
| I-27 | 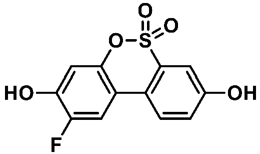 |
| I-28 | 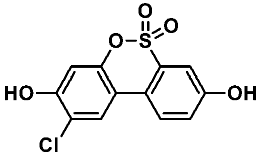 |

In some preferred embodiments, the compound is not
A second aspect of the present invention provides a composition comprising the compound, or salt, stereoisomer or solvate thereof according to the first aspect of the present invention; and a cosmetically acceptable excipient.
A third aspect of the present invention provides a use of the compound, or salt, stereoisomer or solvate thereof according to the first aspect of the present invention, or the composition according to the second aspect of the present invention in the preparation of cosmetics.
A fourth aspect of the present invention provides a method for alleviating skin inflammation, comprising the step of: applying the compound, or salt, stereoisomer or solvate thereof according to the first aspect of the present invention to a subject; or applying the composition according to the second aspect of the present invention to a subject.
A fifth aspect of the present invention provides a method for reducing reactive oxygen species in skin cells, comprising the step of: applying the compound, or salt, stereoisomer or solvate thereof according to the first aspect of the present invention to a subject; or applying the composition according to the second aspect of the present invention to a subject.

Compared with the prior art, the present invention at least has the following advantages:
The present invention provides a class of small molecule compounds having a sulfonic acid or a sulfinic acid lactone structure, which have been verified to have extremely low skin irritation, excellent permeability and high stability, and can not only significantly scavenge reactive oxygen species and reduce oxidative stress, but also alleviate skin inflammation, have excellent ability to protect and repair skin cells and can be used as new raw materials for cosmetics.

It should be understood that, within the scope of the present invention, the above-mentioned technical features of the present invention and the technical features specifically described below (e.g., in the Examples) can be combined with each other, thereby constituting new or preferred technical solutions. Due to space limitations, they will not be described in detail here.

### BRIEF DESCRIPTION OF THE DRAWINGS

One or more embodiments are described by way of example in the accompanying drawings corresponding thereto, and these exemplary descriptions do not constitute a limitation on the embodiments.
FIG. 1 is a behavioral trajectory diagram of a representative zebrafish selected from each group according to an example of the present invention;
FIG. 2 is a statistical graph of the total movement distance of each group of zebrafish within 20 minutes according to an example of the present invention;
FIG. 3 is a statistical graph of the average movement speed of each group of zebrafish within 20 minutes according to an example of the present invention;
FIG. 4 is a representative diagram of staining of inflammatory cells at the nerve thalamus of each group of zebrafish according to an example of the present invention;
FIG. 5 is a statistical diagram of the number of inflammatory cells at the nerve thalamus of each group of zebrafish according to an example of the present invention;
FIG. 6 shows the effect of I-8 on wound repair in zebrafish according to an example of the present invention;
FIG. 7 is a statistical diagram of the caudal fin area of each group of zebrafish according to an example of the present invention;
FIG. 8 is a diagram showing the effect of I-8 on the number of inflammatory cells at the wound of each group of zebrafish according to an example of the present invention;
FIG. 9 is a statistical diagram of the number of inflammatory at the wound site of each group according to an example of the present invention;
FIG. 10 shows the ROS content in zebrafish according to an example of the present invention, wherein the white line area is zebrafish egg yolk (ROS quantitative area), and the values in parentheses are I-8 concentration and unit µg/mL;
FIG. 11 shows statistics of ROS content in zebrafish according to an example of the present invention;
FIG. 12 is a picture of β-galactosidase activity in zebrafish according to an example of the present invention; after the β-galactosidase is dyed, the tissue appears blue-green; compared with the tissue pointed by the arrow on the dorsal fin, the greater the number of arrows indicates that the deeper the blue-green, and the higher the activity of the β-galactosidase;
FIG. 13 shows the statistics of β-galactosidase activity in zebrafish according to an example of the present invention;
FIG. 14 shows the statistics of the expression of anti-oxidative anti-aging related gene in zebrafish according to an example of the present invention;
FIG. 15 shows that compound I-8 is capable of downregulating *TNF-α* in HaCaT according to an example of the present invention;
FIG. 16 shows that compound I-8 is capable of downregulating *IL-8* in HaCaT according to an example of the present invention;
FIG. 17 shows that compound I-8 is capable of downregulating *IL-1β* in HaCaT according to an example of the present invention;
FIG. 18 shows that compound I-8 is capable of downregulating EP2 in HaCaT according to an example of the present invention;
FIG. 19 shows that compound I-8 is capable of downregulating *TRPV1* in HaCaT according to an example of the present invention;
FIG. 20 shows that compound I-8 scavenges ROS according to an example of the present invention;
FIG. 21 shows that compound I-8 enhances the secretion of type I collagen according to an example of the present invention;
FIG. 22 shows that compound I-8 upregulates *COL3A1* gene expression according to an example of the present invention;
FIG. 23 shows that compound I-8 upregulates *COL4A1* gene expression according to an example of the present invention;
FIG. 24 shows that compound I-8 upregulates *COL7A1* gene expression according to an example of the present invention;
FIG. 25 shows that compound I-8 upregulates *LAMA5* gene expression according to an example of the present invention.

### DETAILED DESCRIPTION OF THE INVENTION

Due to the poor stability of traditional antioxidants used in cosmetics, they are subject to stringent application requirements-for example, they degrade when exposed to light during the day and can only be applied at night; they also have poor skin permeability, often requiring high concentrations and large doses to be effective, and often show no effect on some individuals. Furthermore, these antioxidants are highly irritating, requiring strict dosage control for those with sensitive skin, otherwise they can easily trigger severe allergic and inflammatory reactions. Through extensive and in-depth research, the present inventors have developed a class of small molecule polyphenolic compounds having sulfonic acid or sulfinic acid lactone structure. These compounds exhibit low irritation, good permeability, high stability, and can significantly quench reactive oxygen species (ROS), scavenge harmful free radicals, eliminate oxidative stress, alleviate skin inflammation, and soothe and repair damaged skin cells. This class of compounds, and salts, stereoisomers, or solvates thereof, can serve as novel cosmetic raw materials with the potential to replace existing antioxidants.

### Compound

The present invention relates to a class of small molecule compounds having sulfonic acid or sulfinic acid lactone structure, the structure of which is represented by the following general formula I:

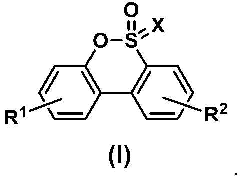

In the above general formula I, X is oxygen or absent. Based on the beneficial effects of further improving the stability and permeability of the compound, X is oxygen.

### R¹ and R²

The number of R¹ is 0-4, such as 0, 1, 2, 3 or 4. As R¹, it can be hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, -OC(O)R¹⁻³, - OCH₂OC(O)R¹⁻⁴, halogen, cyano, nitro, -C(O)R¹⁻⁵ or -COO R¹⁻⁶. In addition, R¹⁻¹, R¹⁻², R¹⁻³, R¹⁻⁴, R¹⁻⁵ and R¹⁻⁶ are each independently hydrogen, C₁₋₆ alkyl, halogen-substituted C₁₋₆ alkyl or -NR^{a}R^{b}; R^{a} and R^{b} are independently hydrogen, C₁₋₆ alkyl or halogen-substituted C₁₋₆ alkyl. In a preferred embodiment, R¹ is hydrogen, hydroxy, C₁₋₆ alkyl, C₁₋₆ alkoxy, amino, - N-C(O)R¹⁻¹, -NR^{a}R^{b}, -CH₂C(O)OR¹⁻², phenyl, -OC(O)R¹⁻³ or -OCH₂OC(O)R¹⁻⁴.

Similarly, the number of R² is 0-4, such as 0, 1, 2, 3 or 4. As R², it can be hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, - OC(O)R¹⁻³, -OCH₂OC(O)R¹⁻⁴, halogen, cyano, nitro, -C(O)R¹⁻⁵ or -COO R¹⁻⁶. In addition, R¹⁻¹, R¹⁻², R¹⁻³, R¹⁻⁴, R¹⁻⁵ and R¹⁻⁶ are each independently hydrogen, C₁₋₆ alkyl, halogen-substituted C₁₋₆ alkyl or -NR^{a}R^{b}; R^{a} and R^{b} are independently hydrogen, C₁₋₆ alkyl or halogen-substituted C₁₋₆ alkyl. In a preferred embodiment, R is hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, -OC(O)R¹⁻³ or - OCH₂OC(O)R¹⁻⁴.

In a more preferred embodiment of the present invention, R¹⁻³ mentioned above is methyl, ethyl, n-propyl, isopropyl, n-butyl, isobutyl, tert-butyl or 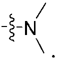 More preferably, R¹⁻³ is isopropyl, tert-butyl or 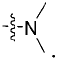 R¹⁻⁴ is methyl, ethyl, n-propyl, isopropyl, n-butyl, isobutyl, tert-butyl, or 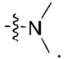 More preferably, R¹⁻⁴ is tert-butyl.

The number of R¹ and R² may be equal or unequal, and in a preferred embodiment, the number of R¹ and R² is equal.

The substitution positions of R¹ and R² are not limited, but in a preferred embodiment, the positions of R¹ and R² are respectively positions 4 and 4' of the biphenyl ring (as shown in I-8 in a specific embodiment of the present invention). As used in the present invention, the position numbering rule of the biphenyl ring is: 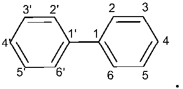 In addition, the O atom in the sulfonic acid or sulfinic acid lactone structure is covalently linked to the carbon at 2', and the S atom is covalently linked to the carbon at 2, and the oxygen atom and the sulfur atom are covalently bonded to each other to form the lactone structure.

Based on the excellent effects of the compound in promoting the scavenging of reactive oxygen species and alleviating inflammation, in a more preferred embodiment, at least one of R¹ and R² has the ability to donate electrons to the parent ring, and preferably both have the ability to donate electrons to the target. For example, R¹ and R² are each independently hydroxy, C₁₋₆ alkoxy, or electron-donating group shown as 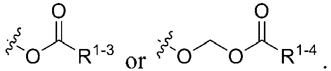 More preferably, R¹ and R² are hydroxy, methoxy, ethoxy, n-propoxy, isopropoxy, n-butoxy, isobutoxy, tert-butoxy, 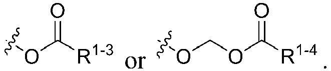 In a specific embodiment of the present invention, R¹ is hydroxy, methoxy, 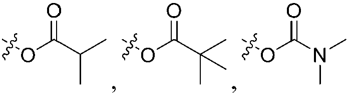 or 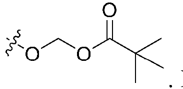 In a specific embodiment of the present invention, R² is hydroxy, methoxy,

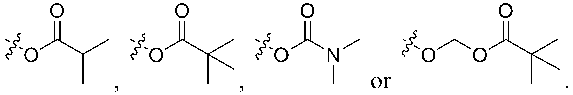

In some specific embodiments of the present invention, the specific structures of the compounds are shown in the following table, wherein compounds I-1 ~ I-12 and I-17 and I-18 have better permeability compared to I-13 ~ I-16.

**TABLE**

| Name | Structural Formula |
|---|---|
| I-1 | 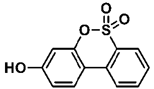 |
| I-2 | 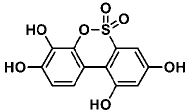 |
| I-3 | 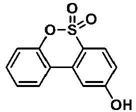 |
| I-4 | 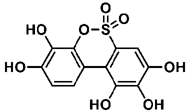 |
| I-5 | 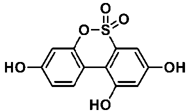 |
| I-6 | 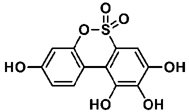 |
| I-7 | 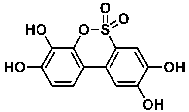 |
| I-8 | 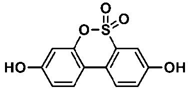 |
| I-9 | 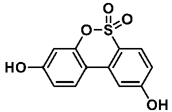 |
| I-10 | 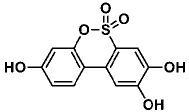 |
| I-11 | 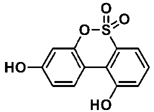 |
| I-12 | 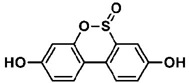 |
| I-13 | 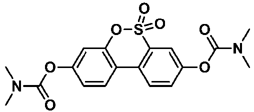 |
| I-14 | 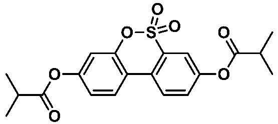 |
| I-15 | 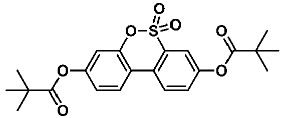 |
| I-16 | 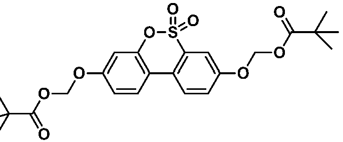 |
| I-17 | 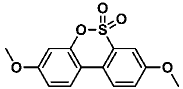 |
| I-18 | 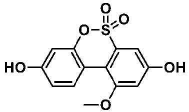 |
| I-19 | 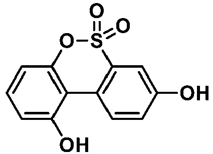 |
| I-20 | 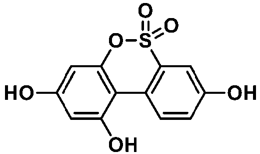 |
| I-21 | 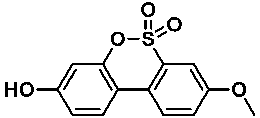 |
| I-22 | 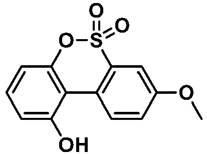 |
| I-23 | 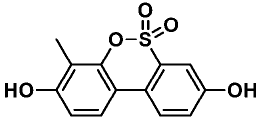 |
| I-24 | 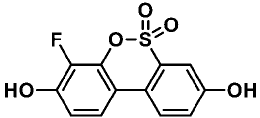 |
| I-25 | 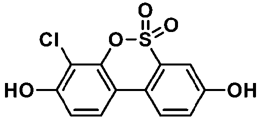 |
| I-26 | 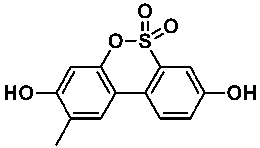 |
| I-27 | 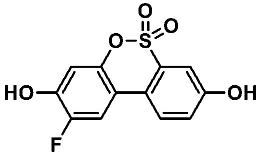 |
| I-28 | 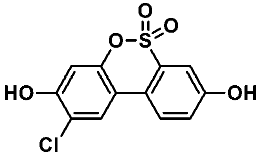 |

### Composition

The present invention also relates to a composition comprising the compound, or salt, stereoisomer, solvate thereof of the present invention. The composition of the present invention may be a cosmetic composition or a topical formulation.

As used in the present invention, the term "cosmetic" refers to a product applied to the surface of the human body (such as the epidermis, hair, lips, etc.) by smearing, spraying or other similar methods to cleanse, care for, beautify or eliminate unpleasant odors, and the product has a soothing effect on the application site.

When used as a cosmetic composition, the composition of the compound, or salt, stereoisomer or solvate thereof of the present invention is used as a cosmetic benefit agent in a cosmetic composition, and further, the composition comprises a cosmetically acceptable medium for diluting, dispersing a cosmetic benefit agent, or using as a carrier thereof in order to facilitate its distribution when the composition is applied to the skin.

These media may be aqueous, anhydrous, or emulsions. Oily carriers, in the presence of water and an emulsifier, form an emulsion system that serve as carriers. Preferably, the composition is an aqueous or emulsion, in particular an water-in-oil or oil-in-water emulsion, preferably an oil-in-water emulsion. Cosmetic compositions are typically in the form of, but not limited to, liquid agents, creams or emulsions. In a preferred embodiment, water is used as a carrier to form the cosmetic composition. In a preferred embodiment, the cosmetic composition further comprises a carrier other than water, such as oil, grease, waxy oil-based matrices (such as coconut oil, palm oil, olive oil, castor oil, mink oil, snake oil, silicone oil and its derivatives, tallow, lanolin and derivatives thereof, carnauba wax, spermaceti wax, beeswax, liquid paraffin, petrolatum, microcrystalline wax, squalane, fatty acids, fatty alcohols and esters, etc.), powdered matrices (such as talc, kaolin, zinc white powder, titanium white powder, bentonite, magnesium stearate, zinc stearate, calcium carbonate, magnesium carbonate, calcium hydrogen phosphate, etc.), and solvent matrices (such as alcohols, small molecule ketones, ethers, small molecule esters).

In the cosmetic composition of the present invention, a cosmetically acceptable excipient may also be added. The term "cosmetically acceptable excipient" refers to a substance that functions to form, stabilize or impart color, fragrance, and other properties of the cosmetic product. For example, preservatives (such as benzoic acid and derivatives thereof, trichlorotert-butanol, chloroxylenol, salicylic acid and derivatives thereof, sorbic acid and derivatives thereof, imidazolidinyl urea, phenylethanol, etc.), antioxidants (such as butyl hydroxyanisole, tert-butyl hydroxyanisole, vitamin E, propyl gallate, etc.), humectants (such as glycerol, propylene glycol, sorbitol, polyethylene glycol, lactic acid, sodium lactate, sodium pyrrolidone carboxylate, hyaluronic acid, hydrolyzed collagen, chitin and derivatives thereof, glucose esters), sunscreen agents (titanium white powder, zinc oxide, aminobenzoate and derivatives thereof, salicylic acid and derivatives thereof, benzophenone, etc.), surfactants (lecithin, saponins, alkyl glycosides, etc.), antioxidants, colorants (such as, organic synthetic pigments such as azo and anthraquinone pigments, etc., or an inorganic pigment such as zinc oxide, titanium dioxide, ferric oxide, ferrous hydroxide, chromium trioxide, ferroferric oxide, etc., or natural pigments such as capsanthin, sorghum red, anthocyanins, etc.), fragrances (natural or blended fragrances such as ambergris, castoreum, musk, civet, rose oil, peppermint oil, spearmint oil, lavender oil, and fennel oil, etc.), water-soluble polymers, chelating agents (such as disodium ethylenediamine tetraacetate and derivatives thereof), and film-forming agents (such as polyvinyl acetate, polyacrylate emulsions, hyaluronic acid, and polyglycerol-2 isostearate, etc.), and the like.

In the cosmetic composition of the present invention, the addition amounts of each raw material and excipient are not limited.

### Use of Compound or Composition

The present invention also relates to a use of the above compound, or salt, stereoisomer or solvate thereof, or composition containing the above compound, or salt, stereoisomer or solvate thereof, for: (i) preparing cosmetics; (ii) alleviating skin inflammation; (iii) reducing reactive oxygen species in skin cells; and/or (iv) repairing damaged skin.

As used herein, the term "skin" includes the skin on the face, neck, chest, back, arms, armpits, arms, hands, legs, and scalp. As used herein, a cosmetic benefit agent refer to components that (a) improve facial or physical characteristics such as skin characteristics after topical application, (b) are beneficial to facial or physical characteristics such as skin features, or (c): (a) and (b). In a preferred embodiment, the compound or cosmetic composition is used for topical application.

### Methods for alleviating skin inflammation and/or reducing reactive oxygen species in skin cells and/or repairing damaged skin

The present invention also relates to a method for (a)alleviating skin inflammation and/or (b) reducing reactive oxygen species in skin cells and/or (c) repairing damaged skin, comprising the steps of applying the above compound, or a salt, stereoisomer or solvate thereof to a subject; or, applying the above composition to a subject. The application way is a non-intestinal application; preferably, external use, such as by smearing or applying topically to the skin, mucous, etc. in an appropriate amount.

### Terminology

As used herein, the term "alkyl" refers to a linear or branched, saturated monovalent hydrocarbon group, wherein the alkyl may optionally be substituted with one or more substituents. In specific embodiments, the alkyl is a linear saturated monovalent hydrocarbon group having 1 to 20 (C₁₋₂₀), 1 to 15 (C₁₋₁₅), 1 to 12 (C₁₋₁₂), 1 to 10 (C₁₋₁₀), or 1 to 6 (C₁₋₆) carbon atoms, or a branched saturated monovalent hydrocarbon group having 3 to 20 (C₃₋₂₀), 3 to 15 (C₃₋₁₅), 3 to 12 (C₃₋₁₂), 3 to 10 (C₃₋₁₀), or 3 to 6 (C₃₋₆) carbon atoms. The linear C₁₋₆ and branched C₃₋₆ alkyl as used herein are also referred to as "lower alkyl". Examples of alkyl group include, but are not limited to, methyl, ethyl, propyl (including all isomeric forms), n-propyl, isopropyl, butyl (including all isomeric forms), n-butyl, isobutyl, tert-butyl, pentyl (including all isomeric forms) and hexyl (including all isomeric forms). For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the alkyl is an optionally substituted alkyl described elsewhere herein. In some embodiments, the C₁₋₆ alkyl is C₁₋₄ alkyl, and the C₁₋₄ alkyl is methyl, ethyl, n-propyl, isopropyl, n-butyl, isobutyl or tert-butyl. In other embodiments, any one or more hydrogen atoms in the C₁₋₆ alkyl are substituted by halogen. In other embodiments, the halogen-substituted C₁₋₆ alkyl is a halogen-substituted C₁₋₄ alkyl. In other embodiments, the halogen-substituted C₁₋₄ alkyl is a halogen-substituted methyl, a halogen-substituted ethyl, a halogen-substituted n-propyl, a halogen-substituted isopropyl, a halogen-substituted n-butyl, a halogen-substituted isobutyl, or a halogen-substituted tert-butyl.

As used in the present invention, the term "alkoxy" refers to a stable linear or branched, or cyclic hydrocarbyl group, or a combination thereof, consisting of the indicated number of carbon atoms and one or more (one to three in one embodiment) O atoms. Examples of alkoxys include, but are not limited to, -O-CH₃, -O-CH₂-CH₃, -O-CH₂-CH₂-CH₃, -O-CH-(CH₃)₂, and - O-CH₂-CH₂-O-CH₃. In one embodiment, the alkoxy is an optionally substituted alkoxy as described elsewhere herein. In some embodiments, the alkoxy is a C₁₋₆ alkoxy. In some embodiments, the alkoxy is a C₁₋₄ alkoxy. In some embodiments, the alkoxy is methoxy, ethoxy, n-propoxy, isopropoxy, n-butoxy, isobutoxy or tert-butoxy. In other embodiments, any one or more of the C₁₋₆ alkoxys is substituted by halogen. In other embodiments, the halogen-substituted C₁₋₆ alkoxy is a halogen-substituted methoxy, a halogen-substituted ethoxy, a halogen-substituted n-propoxy, a halogen-substituted isopropoxy, a halogen-substituted n-butoxy, a halogen-substituted isobutoxy, or a halogen-substituted tert-butoxy.

As used herein, the term "halogen" is fluorine, chlorine, bromine, or iodine.

As used herein, "(O)" is an =O structure, such as -NH-C(O)- is an amide group, -OC(O)-is an acyloxy, -C(O)- is a carbonyl.

As used herein, the term "hydrogen" includes protons (1H), deuterium (2H), tritium (3H), and/or mixtures thereof. In the compound described herein, the one or more hydrogen-occupied positions may be enriched by deuterium and/or tritium. Such isotopically enriched analogs can be prepared from suitable isotopically labeled starting materials available from commercial sources or prepared by known literature steps.

As used herein, the term "hydroxy" refers to -OH.

As used herein, the term "amino" refers to -NH₂.

As used herein, the term "cyano" refers to -CN.

As used herein, the term "nitro" refers to -NO₂.

The term "substituted" refers to a moiety having a substituent that replaces hydrogen or one or more non-hydrogen atoms on the molecule.

As used herein, the term "solvate" refers to a compound formed by the interaction of a solvent with a compound or a salt thereof provided by the present invention.

In order to make the objectives, technical solutions and advantages of the embodiments of the present invention clearer, the present invention is further described below with reference to specific examples. It should be understood that these examples are only used to illustrate the present invention and are not intended to limit the scope of the present invention. The experimental methods without specific conditions in the following examples are usually according to conventional conditions, or according to the conditions recommended by the manufacturer. Unless otherwise specified, percentages and parts are weight percentages and parts by weight. Experimental materials and reagents used in the following examples are available from commercially available channels, unless otherwise specified.

Unless otherwise specified, the technical and scientific terms used herein have the same meaning as commonly understood by one of ordinary skill in the art to which this application belongs, and it should be noted that the terminology used herein is for the purpose of describing specific embodiments only and is not intended to limit the exemplary embodiments of the present application.

### General Synthesis Step I

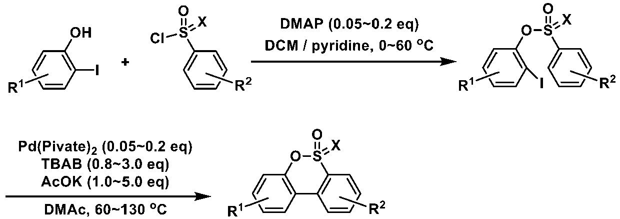

### General Synthesis Step II

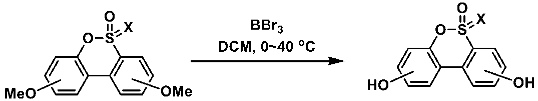

### General Synthesis Step III

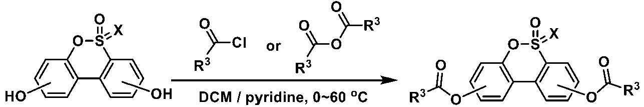

### General Synthesis Step IV

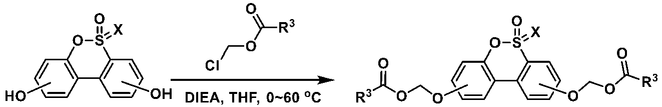

### Example 1: Synthesis and Characterization of Compound I-8

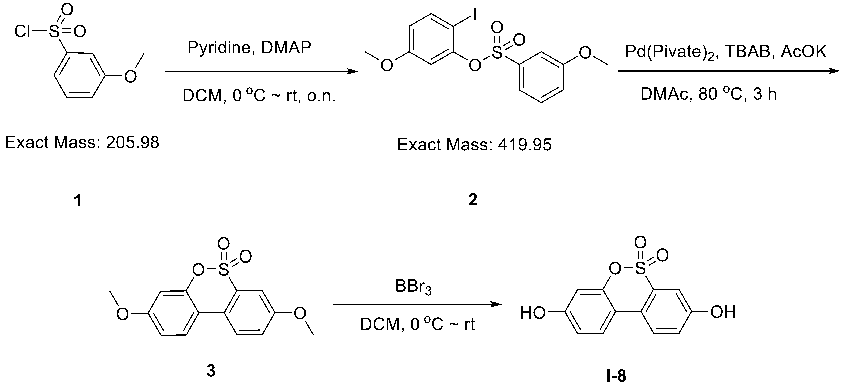

Synthesis of Intermediate **2:** 2-iodo-5-methoxyphenol (0.73 g, 2.92 mmol, 1.2 eq), DMAP (0.03 g, 0.24 mmol, 0.1 eq) were dissolved in DCM (5.00mL) and pyridine (10.00mL), and cooled to 0 °C and stirred until homogeneous, then compound 1(0.50 g, 2.43 mmol, 1 eq) was dissolved in DCM (5.00mL), then the solution was slowly added dropwisely to the reaction system, the temperature was controlled to be 0 °C, the system was naturally warmed to room temperature after dropwise addition was completed, and reacted overnight. LC-MS indicated that the reaction of the raw material was completed, then stopped stirring, the reaction mixture was extracted with water and DCM, and the organic phase was separated, concentrated, and purified by silica gel column chromatography (PE:EA = 10:1) to obtain intermediate **2** (0.96 g, 2.23 mmol, 94.61% yield, 93.42% purity) as a yellow oil. LC-MS: [M+1]⁺=421.

Synthesis of Intermediate **3** (i.e. I**-17**): Compound **2** (0.2 g, 0.48 mmol, 1 eq), Pd (Pivate)₂ (0.015 g, 0.048 mmol, 0.1 eq), TBAB (0.18 g, 0.57 mmol, 1.2 eq), potassium acetate (0.14 g, 1.43 mmol, 3 eq) were dissolved in DMAC(2.00mL), completely replaced with nitrogen, heated to 60 °C, and stirred and reacted under nitrogen protection overnight. LC-MS indicated completion of the reaction, the reaction mixture was filtered, extracted with EA, concentrated, and purified by silica gel column chromatography (PE:EA = 1:1) to obtain Intermediate 3 (0.12 g, 0.42 mmol, 88.49% yield, 93.32% purity) as a white solid. ¹H NMR (400 MHz, Chloroform-d) δ 7.76 (d, J = 2.8 Hz, 1H), 7.74 (d, J = 2.7 Hz, 1H), 7.43 (d, J = 2.7 Hz, 1H), 7.28 - 7.23 (m, 1H), 6.93 (dd, J = 8.8, 2.6 Hz, 1H), 6.84 (d, J = 2.6 Hz, 1H), 3.91 (s, 3H), 3.87 (s, 3H). LC-MS: [M+1]⁺=293.

Synthesis of Compound **I-8:** Compound **3**(1.00 g, 3.42 mmol, 1 eq) was dissolved in DCM (20.00mL), the mixture was cooled to 0 °C, BBr₃ (1.0 M in DCM, 13.68 mmol, 13.68 mL, 4 eq) was slowly added dropwisely under nitrogen protection, the temperature was controlled to be 0 °C, the system was naturally warmed to room temperature after dropwise addition was completed, and reacted overnight. LC-MS indicated completion of the reaction. The reaction was quenched with ice water, extracted with EA and DCM, concentrated, and purified by silica gel column chromatography (PE:EA = 1:1) to obtain Compound **I-8** (0.25 g, 0.95 mmol, 27.78% yield, 100% purity) as a pale yellow solid. 1H NMR (400 MHz, DMSO-d6) δ 7.96 (d, J = 8.5 Hz, 1H), 7.92 (d, J = 8.8 Hz, 1H), 7.27 - 7.22 (m, 2H), 6.87 (dd, J = 8.6, 2.4 Hz, 1H), 6.78 (d, J = 2.4 Hz, 1H). LC-MS: [M-1]⁻=263_{∘}

### Example 2: Synthesis and Characterization of Compound I-11

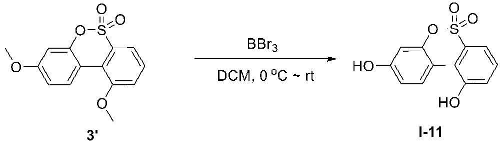

In Example 1, Compound **3'** was also generated from the reaction of Compound **2** to Compound **3.** Compound 3' was subjected to the same demethylation step to obtain Compound **I-11.** LC-MS: [M-1]⁻=263.

### Example 3: Synthesis and Characterization of Compound I-13

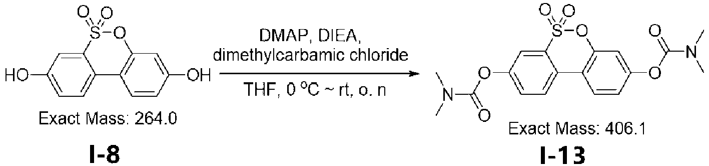

Compound **I-8** (130 mg, 0.49 mmol), DMAP (6 mg, 0.05 mmol) and DIEA (254 mg, 1.97 mmoL) were dissolved in THF (5 mL), the mixture was cooled to 0 °C, then dimethylcarbamoyl chloride (212 mg, 1.97 mmoL) was slowly added dropwise to the reaction system, the temperature was controlled to be 0 °C. After the dropwise addition was complete, the system was warmed naturally to room temperature and reacted overnight. The mixture was concentrated and purified by silica gel column chromatography (DCM:EA = 100:5) to obtain **I-13** (90 mg, yield 45.01%) as a white solid. ¹H NMR (400 MHz, DMSO-*d*₆) δ 8.33 (d, *J =* 8.8 Hz, 1H), 8.30 (d, *J =* 8.8 Hz, 1H), 7.92 (d, *J =* 2.4 Hz, 1H), 7.74 (dd, *J =* 8.7*,* 2.5 Hz, 1H), 7.45 (d, *J =* 2.3 Hz, 1H), 7.34 (dd, *J =* 8.7, 2.4 Hz, 1H), 3.08 (d*, J =* 6.2 Hz, 6H), 2.94 (d, *J =* 2.8 Hz, 6H). LCMS: m/z =407.1 (M+H⁺, ESI).

### Example 4: Synthesis and Characterization of Compound I-14 and I-15

Using the synthesis method in Example 3, **I-8** was also used as a starting material, and dimethylcarbamoyl chloride was replaced with isobutyryl chloride or pivaloyl chloride to obtain **I-14** and **I-15,** which were both white solid. **I-14:** LCMS: m/z =405 (M+H⁺, ESI). **I-15:** ¹H NMR (400 MHz, DMSO-*d*₆) δ 8.24 (d, *J* = 8.8 Hz, 1H), 8.07 (dd, *J* = 7.7, 1.3 Hz, 1H), 7.83 (t, *J =* 8.0 Hz, 1H), 7.75 (dd, *J* = 8.3, 1.3 Hz, 1H), 7.54 (d, *J =* 2.4 Hz, 1H), 7.38 (dd, *J* = 8.8, 2.4 Hz, 1H), 1.35 (s, 9H), 1.32 (s, 9H). LCMS: m/z =433 (M+H⁺, ESI).

### Example 5: Synthesis and Characterization of Compound I-1

According to General Synthesis Steps I and II, and using the synthetic method described in Example 1, compounds 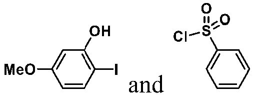 were used as starting materials to obtain Compound **I-1:** LCMS: m/z =247 (M-H⁺, ESI).

### Example 6: Synthesis and Characterization of Compound I-2

According to General Synthesis Steps I and II, and using the synthetic method described in Example 1, compounds 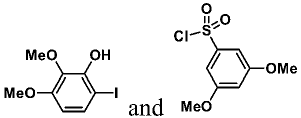 were used as starting materials to obtain Compound **I-2:** LCMS: m/z =295 (M-H⁺, ESI).

### Example 7: Synthesis and Characterization of Compound I-3

According to General Synthesis Steps I and II, and using the synthetic method described in Example 1, compounds 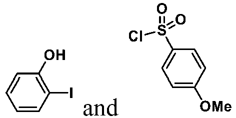 were used as starting materials to obtain Compound **I-3:** LCMS: m/z =247 (M-H⁺, ESI).

### Example 8: Synthesis and Characterization of Compound 1-4

According to General Synthesis Steps I and II, and using the synthetic method described in Example 1, compounds 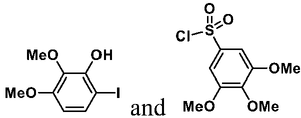 were used as starting materials to obtain Compound **I-1:** LCMS: m/z =311 (M-H⁺, ESI).

### Example 9: Synthesis and Characterization of Compound I-5

According to General Synthesis Steps I and II, and using the synthetic method described in Example 1, compounds 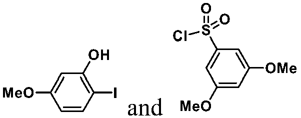 were used as starting materials to obtain Compound **I-5:** LCMS: m/z =279 (M-H⁺, ESI).

### Example 10: Synthesis and Characterization of Compound I-6

According to General Synthesis Steps I and II, and using the synthetic method described in Example 1, compounds 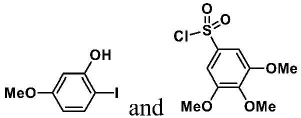 were used as starting materials to obtain Compound **I-6:** LCMS: m/z =295 (M-H⁺, ESI).

### Example 11: Synthesis and Characterization of Compound I-7

According to General Synthesis Steps I and II, and using the synthetic method described in Example 1, compounds 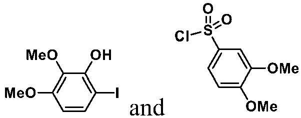 were used as starting materials to obtain Compound **I-7:** LCMS: m/z =295 (M-H⁺, ESI).

### Example 12: Synthesis and Characterization of Compound I-9

According to General Synthesis Steps I and II, and using the synthetic method described in Example 1, compounds 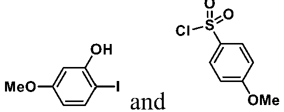 were used as starting materials to obtain Compound **I-9:** LCMS: m/z =263 (M-H⁺, ESI).

### Example 13: Synthesis and Characterization of Compound I-10

According to General Synthesis Steps I and II, and using the synthetic method described in Example 1, compounds 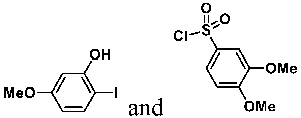 were used as starting materials to obtain Compound **I-10:** LCMS: m/z =279 (M-H⁺, ESI).

### Example 14: Synthesis and Characterization of Compound I-12

According to General Synthesis Steps I and II, and using the synthetic method described in Example 1, compounds 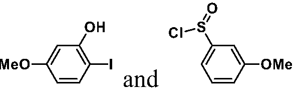 were used as starting materials to obtain Compound **I-12:** LCMS: m/z =247 (M-H⁺, ESI).

### Example 15: Synthesis and Characterization of Compound I-16

According to General Synthesis Step IV, compounds **I-3** and 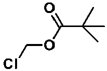 were used as starting materials to obtain Compound **I-16:** LCMS: m/z =493 (M+H⁺, ESI).

### Example 16: Synthesis and Characterization of Compound I-18

According to General Synthesis Step I, compounds 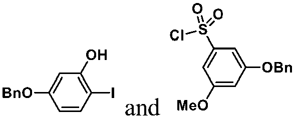 were used as starting materials to obtain Intermediate 4:

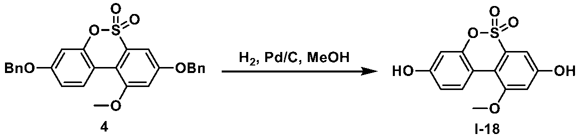

Intermediate **4** was debenzylated by hydrogenation to obtain Compound **I-18:** LCMS: m/z =293 (M-H⁺, ESI).

### Example 17: Synthesis and Characterization of Compound I-19

According to General Synthesis Step I, compounds 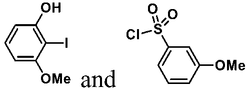 were used as starting materials to obtain Compound **I-19:** ¹H NMR (400 MHz, DMSO-*d*₆) δ10.95 (s, 1H), 10.67 (s, 1H), 8.58 (d, J = 8.9 Hz, 1H), 7.32 - 7.29 (m, 1H), 7.28 - 7.26 (m, 1H), 7.26 - 7.23 (m, 1H), 6.98 (dd, J = 8.4, 1.2 Hz, 1H), 6.90 (dd, J = 8.1, 1.2 Hz, 1H). LCMS: m/z =263 (M-H⁺, ESI).

### Example 18: Synthesis and Characterization of Compound I-20

According to General Synthesis Step I, compounds 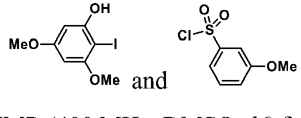 were used as starting materials to obtain Compound **I-20:** ¹H-NMR (400 MHz, DMSO-*d6*) δ 10.77 (s, 1H), 10.46 (s, 1H), 10.22 (s, 1H), 8.45-8.43 (d, 1H), 7.25-7.24 (d, 1H), 7.21-7.18 (dd, 1H), 6.45-6.44 (d, 1H), 6.28-6.27 (d, 1H). LCMS: m/z =279 (M-H⁺, ESI).

### Example 19: Synthesis and Characterization of Compound I-21

According to General Synthesis Step I, compounds 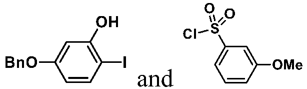 were used as starting materials to obtain Intermediate **5:**

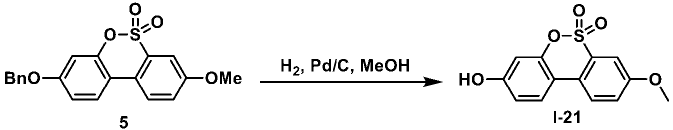

Intermediate **5** was debenzylated by hydrogenation to obtain Compound **I-21:** ¹H-NMR (400 MHz, DMSO-d6) δ 10.48 (s, 1H), 8.07 (d, *J=* 8.7 Hz, 1H), 7.99 (d, *J=* 8.7 Hz, 1H), 7.48 - 7.41 (m, 2H), 6.89 (dd, *J =* 8.7, 2.4 Hz, 1H), 6.81 (d, *J =* 2.4 Hz, 1H), 3.90 (s, 3H). LCMS: m/z =277 (M-H⁺, ESI).

### Example 20: Synthesis and Characterization of Compound I-22

According to General Synthesis Step I, Compounds 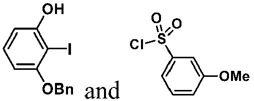 were used as starting materials to obtain Intermediate **6:**

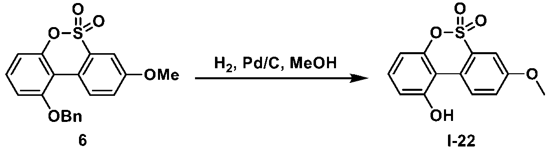

Intermediate **6** was debenzylated by hydrogenation to obtain Compound **I-22:** ¹H-NMR (400 MHz, DMSO-d6) δ 11.04 (s, 1H), 8.67 (d, *J* = 9.0 Hz, 1H), 7.49 (d, *J* = 2.8 Hz, 1H), 7.44 (dd, *J =* 9.1, 2.9 Hz, 1H), 7.30 (t, *J =* 8.2 Hz, 1H), 7.01 (dd, *J =* 8.3, 1.2 Hz, 1H), 6.92 (dd, *J =* 8.1, 1.1 Hz, 1H), 3.91 (s, 3H). LCMS: m/z =277 (M-H⁺, ESI).

### Example 21: Synthesis and Characterization of Compound I-23

According to General Synthesis Step I, compounds 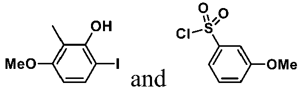 were used as starting materials to obtain Compound **I-23:** LCMS: m/z =277 (M-H⁺, ESI).

### Example 22: Synthesis and Characterization of Compound I-24

According to General Synthesis Step I, compounds 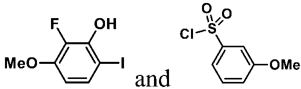 were used as starting materials to obtain Compound **I-24:** LCMS: m/z =281 (M-H⁺, ESI).

### Example 23: Synthesis and Characterization of Compound I-25

According to General Synthesis Step I, compounds 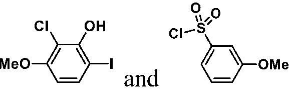 were used as starting materials to obtain Compound **I-25:** LCMS: m/z =297 (M-H⁺, ESI).

### Example 24: Synthesis and Characterization of Compound I-26

According to General Synthesis Step I, compounds 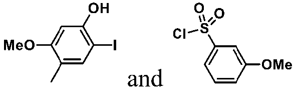 were used as starting materials to obtain Compound **I-26:** LCMS: m/z =277 (M-H⁺, ESI).

### Example 25: Synthesis and Characterization of Compound I-27

According to General Synthesis Step I, compounds 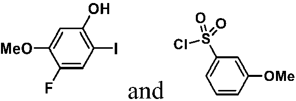 were used as starting materials to obtain Compound **I-27:** LCMS: m/z =281 (M-H⁺, ESI).

### Example 26: Synthesis and Characterization of Compound I-28

According to General Synthesis Step I, compounds 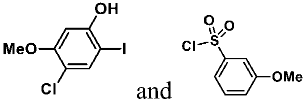 were used as starting materials to obtain Compound **I-28:** LCMS: m/z =297 (M-H⁺, ESI).

### Test Example 1: Evaluation of relieving stinging and relieving anti-inflammatory efficacies of the compound using a zebrafish model

### Experimental animal: Healthy wild-type AB strain zebrafish at 3 dpf (days post fertilization)

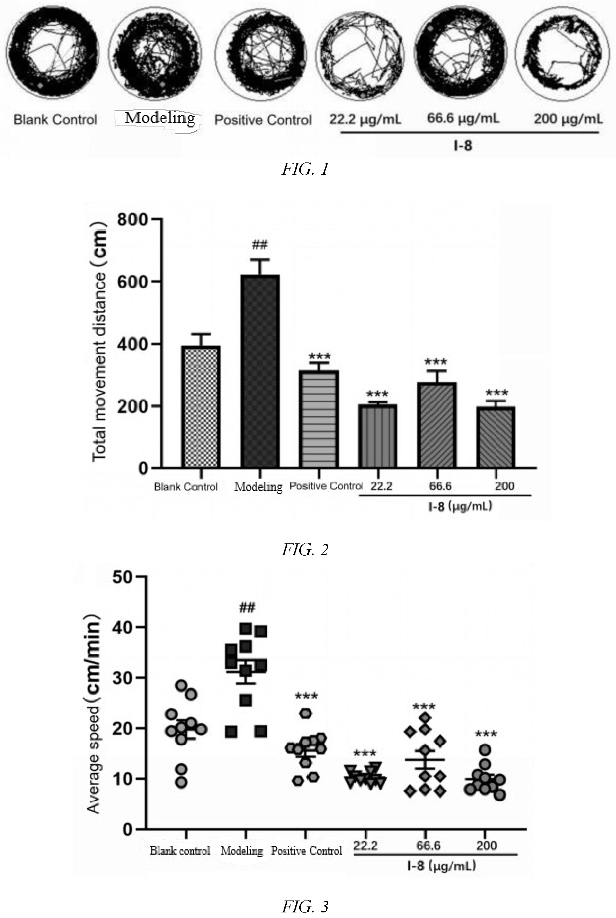

**Evaluation of the relieving stinging efficacy:** Healthy AB strain zebrafish were taken and randomly divided into four groups: blank control, modeling group, positive control, and sample group. The zebrafish in the blank control group were treated with embryo culture water; the zebrafish in the modeling group were treated with a modeling drug (100 µM SDS , sodium dodecyl sulfate); the zebrafish in the positive control group were treated with both the modeling agent and a positive control drug (200 µM asiaticoside solution); and the zebrafish in the sample group were treated with both the modeling drug and different concentrations of the test sample (high, medium, and low concentrations). After continuous treatment for a certain period, the zebrafish's behavioral trajectories were recorded using a behavior analyzer, and the changes in total movement distance and speed were statistically analyzed, the results are shown in FIGS. 2-4. SDS caused stinging in the zebrafish, prompting them to move more vigorously, and therefore, compared with the blank control group, the total movement distance and average speed of the modeling group were significantly increased. In the behavioral trajectory diagram in FIG. 2, the black lines represent the zebrafish's behavioral trajectories in the hole; the more numerous and chaotic the lines, the more vigorous the movement of the zebrafish.

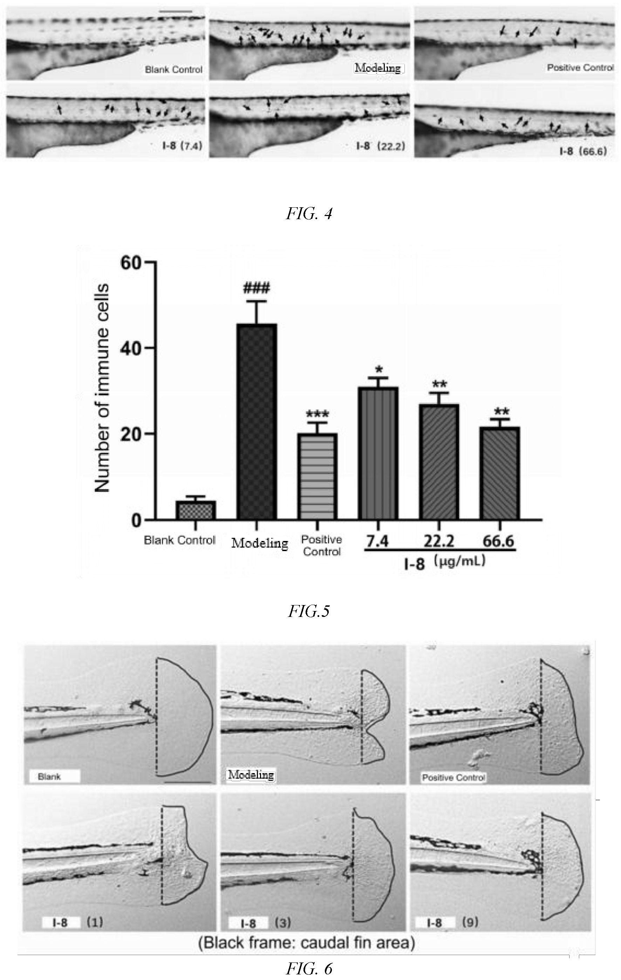

**Evaluation of the relieving anti-inflammatory efficacy:** As described above, the modeling drug was 40 µM copper sulfate, the positive control drug was 200 µM asiaticoside, and the concentrations of **I-8** were 66.6, 22.2, and 7.4 µg/mL respectively. After treating zebrafish as described above, inflammatory cells in their bodies were stained, the number of inflammatory cells at the neurothalamus was counted, and statistical analysis was performed. The results are shown in FIGS. 5 and 6.

**Conclusions: I-8** exhibits certain relieving stinging efficacy when its effective concentrations are 22.2, 66.6, and 200 µg/mL. **I-8** exhibits certain relieving anti-inflammatory efficacy when its effective concentrations are 7.4, 22.2, and 66.6 µg/mL.

### Test Example 2: Evaluation of the permeability of the compound into skin using a Franz diffusion cell

Experimental materials: skin from the back of a one-month-old Bama miniature pig, 0.8-1.0 mm thick; 0.1 mg/mL solution of the compound to be test, dissolvent: 50 mM Sodium Phosphate Buffer (PB) buffer solution containing 20% ethanol.

Experimental procedure:
1. 400 uL solution of the compound to be test was pipetted into the feed cell, repeated three times.
2. About 7600 uL of 50 mM PB buffer solution was added to the corresponding receiving cell.
3. The compound to be test was permeated at 32 °C for 2 hrs and 6 hrs.
4. At 2 hrs and 6 hrs, the skin was washed with 50 mM PB buffer solution containing 20% ethanol, the skin from the permeation area was removed and homogenized, and acetonitrile solution containing internal standard was added.
5. Centrifugation was performed, 100 µL of the supernatant was taken and added into 100 µL of purified water, after mixing uniformly, quantitative analysis was performed by LC-MS/MS.

The quantitative detection results of the permeability of the compounds in the examples into the skin are shown in Table 2 below:

**TABLE 2**

| Compounds | Time (h) | Concentration of the compound to be test in the skin (ng/g) | | | | | |
|---|---|---|---|---|---|---|---|
| | | Skin-1 | Skin-2 | Skin-3 | Mean | SD | CV (%) |
| **I-8** | 2 | 7450 | 6600 | 7450 | 7167 | 491 | 6.8 |
| | 6 | 13650 | 10450 | 10150 | 11417 | 1940 | 17.0 |
| **I-14 (I-8 detected)** | 24 | 13550 | 7150 | 14300 | 11667 | 3929 | 33.7 |
| **I-13 (I-8 detected)** | 24 | 368 | 340 | 273 | 327 | 49 | 14.9 |

Compound **I-8** has a good skin permeability, and can achieve a permeation amount of 10 ug/g within 6 hours.

### Test Example 3: Evaluation of repair efficacy of the compound using a zebrafish model

### Experimental animal: Healthy wild-type AB strain zebrafish at 3 dpf (days post fertilization)

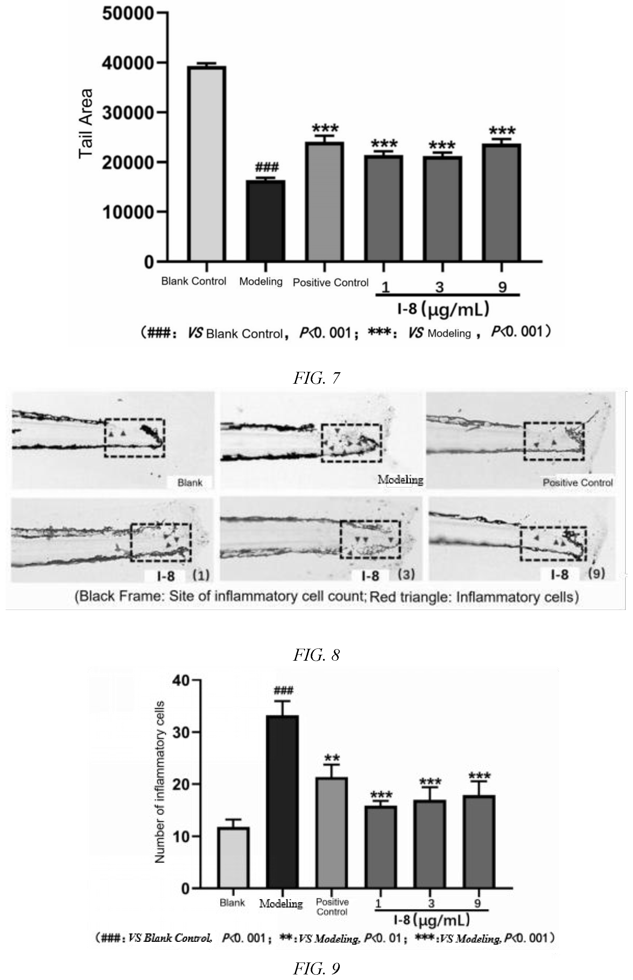

**Evaluation of wound repair efficacy:** Healthy AB strain zebrafish were taken as experimental animal, and randomly divided into blank control group, modeling group, positive control group, and sample group. The zebrafish in the blank control group were treated with culture water; the zebrafish in the modeling group were treated with culture water after cutting the caudal fin; the zebrafish in the positive control group and sample group were treated with 600 µg/mL hyaluronic acid and **I-8** with different concentrations (1, 3, 9 µg/mL) respectively after cutting the caudal fin. The larva fish in above groups were placed in a constant temperature incubator at 28.5 °C and kept in continuous darkness for 48 hrs, with the culture medium changed once every 24 hrs. Image-Pro Plus software was used to calculate the area of the caudal fin of each group of zebrafish. The data were statistically analyzed using GraphPad software. The results are shown in FIGS. 7-8.

**Evaluation of inflammatory repair efficacy:** As described above, the larva fish in each group were placed in a constant temperature incubator at 28.5 °C and kept in continuous darkness for 48 hrs. Inflammatory cells in the fish body were labeled with neutral red, and the number of inflammatory cells at the broken tail was counted. The data were statistically analyzed using GraphPad software. The results are shown in FIGS. 9-10.

**Conclusions:** As shown in FIGS. 7-8, compared with the blank control group, although the caudal fin of the zebrafish in the modeling group regenerated, its area was significantly reduced. When **I-8** at concentrations of 1, 3, and 9 µg/mL was applied to the zebrafish tail amputation model respectively, the caudal fin area of the zebrafish increased compared with the modeling group, and this difference has a statistical significance. The result indicates that when the action concentration of **I-8** is 1, 3, and 9 µg/mL, it can further promote the regeneration of the caudal fin tissue, and has a certain wound repair efficacy. As shown in FIGS. 9-10, compared with the blank control group, the number of inflammatory cells at the broken tail of the zebrafish in the modeling group was significantly increased. When **I-8** at concentrations of 1, 3, and 9 µg/mL was applied to the zebrafish tail amputation model respectively, the number of inflammatory cells at the wound decreased compared with the modeling group, and this difference has a statistical significance. This result suggests that when the action concentration of **I-8** is 1, 3, and 9 µg/mL, it has a certain inflammation repair efficacy.

### Test Example 4: Kinetic solubility in aqueous buffer compared with Urolithin A

**Experimental Methods:** 30 µL of the 10 mM DMSO stock solution of the compound to be test was placed in a solubility sample plate (duplicate), 970 µL of aqueous buffer solution was added to the plate, and the plate was sealed with a sealing membrane and shaken at 1100 rpm for 2 hrs at 25 °C. The sample in the solubility plate was transferred to a filter plate, and filtered to obtain a filtrate. 10 µL of the filtrate was transferred into 980 µL of methanol along with 10 µL of DMSO, and then the solution was diluted 10-fold with methanol:water (1:1), and the resulting sample was analyzed by LC-MS/MS to obtain Area (filtered). Separately, 10 mM of the DMSO stock solution of the compound to be test was diluted to 300 µM with DMSO. 10 µL of this 300 µM DMSO solution and 10 µL of aqueous buffer solution were added into 980 µL of methanol, and then diluted 10-fold with methanol:water (1:1), and the resulting sample was analyzed by LC-MS/MS to obtain Area (std). The kinetic solubility was calculated according to the following formula: $Solubility \left(μM\right) = \frac{Are {a}_{filtered} \times D {F}_{filtered}}{Are {a}_{std} \times D {F}_{std}} \times \left[std\right] \times D {F}_{std}$ wherein DF is the dilution factor.

| **Compound** | **Kinetic Solubility (µM)** |
|---|---|
| **Urolithin A** | 98.76 |
| **I-8** | > 300* |

*Accurate kinetic solubility values cannot be obtained when the actual solubility is greater than 300 µM.

**Conclusions:** Compared with the comparative compound **Urolithin A, I-8** has better solubility, overcoming the problem that **Urolithin A** has poor water solubility and is difficult to develop into topical formulations.

### Test Example 5: Evaluation of the antioxidant efficacy of the compound using a zebrafish model

### Experimental animal: Healthy wild-type AB strain zebrafish at 48 hpf (hours post-fertilization)

### Evaluation of the antioxidant efficacy:

Healthy AB strain zebrafish were taken as experimental animal. A zebrafish oxidative aging model was constructed using chemical mutagenesis with 4 µM menadione as the modeling drug. Fucoxanthin was used as a positive control compound. After treating the zebrafish oxidative aging model with different concentrations of **I-8** for 22 hrs, changes in the reactive oxygen species (ROS) content, β-galactosidase activity, and the expression of three antioxidant and anti-aging related genes (Cu/Zn-sod, Mn-sod, and ampk) were respectively detected and statistically analyzed.

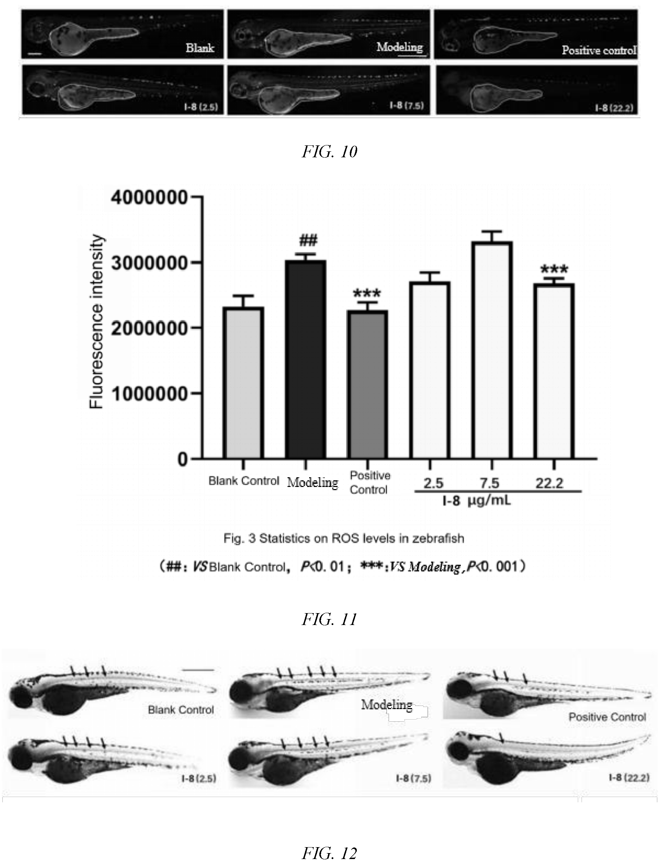

During the experiment, the modeling drug, positive compound, and **I-8** were dispersed together in the culture water of zebrafish. The larvae fish in each group were placed in a constant temperature incubator at 28.5 °C and continuously treated in the dark for 22 hrs. Cell ROX^{™} Deep Red fluorescent probes and β-galactosidase staining kits were used to specifically label ROS and β-galactosidase in the fish, respectively, and the staining intensity was quantified using Image-Pro Plus software. After extracting RNA from the whole fish, the expression changes of three genes (Cu/Zn-sod, Mn-sod, and ampk) in the fish were detected at the mRNA level using Quantitative Real-time PCR (RT-qPCR). The data were statistically analyzed using GraphPad software. The results are shown in FIGS. 10-14.

**Conclusions:** As shown in FIGS. 10-13, compared with the blank control group, after continuously treating the zebrafish with menadione for 22 hrs, the ROS content and β-galactosidase activity in zebrafish were significantly increased, and this difference has a certain statistical significance. Since menadione is an oxidant, it can generate unstable semiquinones through the intracellular reductase system, thereby leading to the production of large amounts of ROS and accelerating aging of the body, and β-galactosidase is a hydrolytic enzyme in cytolysosome, and its increased activity is a significant characteristic of cellular aging. When the zebrafish oxidative aging model was treated with **I-8** at a concentration of 22.2 µg/mL, compared with the modeling group, both the ROS content and β-galactosidase activity in the fish were downregulated, and this difference has a statistical significance. These results indicates that **I-8** at a concentration of 22.2 µg/mL has a certain antioxidant effect.

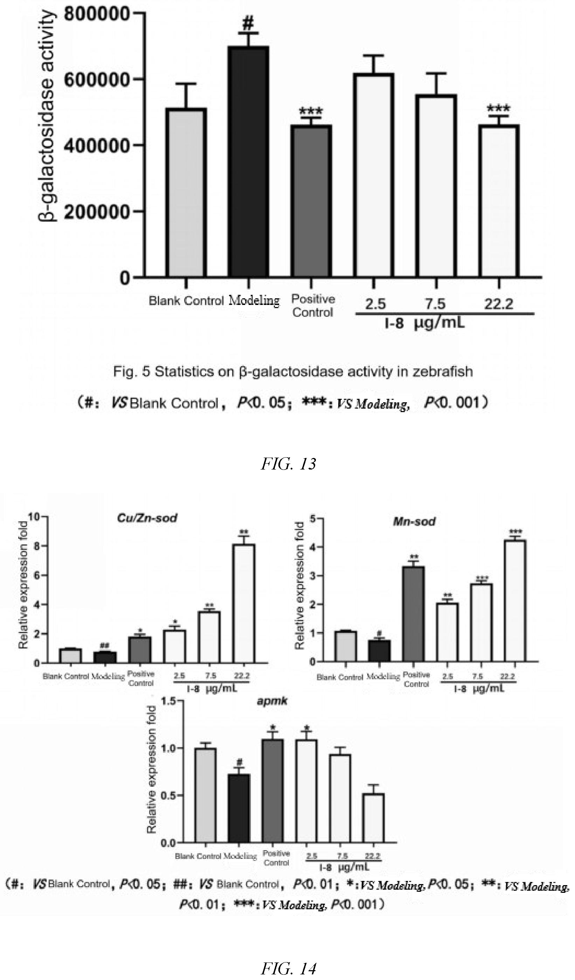

As shown in FIG. 14, when zebrafish oxidative aging models were treated with **I-8** at concentrations of 2.5, 7.5, and 22.2 µg /mL respectively, the expression of both Cu/Zn-sod and Mn-sod genes was upregulated compared to the modeling group, and this difference has a statistical significance. When the zebrafish oxidative aging model was treated with **I-8** at a concentration of 2.5 µg/mL, the expression of the ampk gene was upregulated compared to the modeling group, and this difference has a statistical significance.

The above results indicate that when the active concentration of **I-8** is 2.5, 7.5, and 22.2 µg/mL, the abnormal expression of the Cu/Zn-sod and Mn-sod genes induced by menadione can be reversed; and when the active concentration is 2.5 µg/mL, the abnormal expression of the ampk gene induced by menadione can be reversed.

Inflammation is a physiological response that protects the body from various injuries such as physical injury, pathogens, toxic chemicals exposure and ultraviolet irradiation. Early inflammation is mainly manifested as capillary expansion, hypertonicity and edema. Various inflammatory mediators play key roles in the course of acute and chronic inflammation, such as interleukin-1α (interleukin-1β, IL- 1β), interleukin-8 (IL-8), tumor necrosis factor-α (TNF-α) and prostaglandin E2 (PGE2), etc. PGE2 is the most abundant prostaglandin produced in the body, which is originated from arachidonic acid. Arachidonic acid is first converted into prostaglandin H2 (PGH2) by cyclooxygenase (COX), and then further catalyzed by prostaglandin E synthase to generate PGE2. Finally, PGE2 exerts its predominantly inflammatory biological functions by acting on four E-type prostaglandin (EP) receptors, EP1-4. Among them, all EP2 receptors are coupled to Gs proteins and primarily signal via the cAMP-PKA-CREB pathway triggered by adenylate cyclase. Studies have also proved that selective small-molecule antagonists targeting EP2 receptors can be developed to alleviate downstream pathological processes mediated by EP2 receptors, thus enabling the development of next-generation anti-inflammatory therapies.

Furthermore, the TRPV1 receptor is a nociceptor that can be activated by various factors such as chemical substances (capsaicin), noxious heat stimulation, and acidification. TRPV1 is widely present in class C sensory nerve afferent fibers and keratinocytes. When the TRPV1 receptor is activated, monovalent and divalent cations (mainly Ca²⁺) enter the cell, triggering an action potential that is transmitted to higher central nervous system, resulting in a burning pain sensation. Therefore, blocking or inhibiting the expression of TRPV1 receptor protein after treatment with a test substance can help alleviate the burning pain and achieve a soothing effect. Thus, the expression level of the TRPV1 gene in keratinocytes after sample treatment can be detected as a preliminary indicator to determine whether the sample has a soothing effect.

In the following Test Examples 6-1 and 6-2, using immortalized keratinocytes (HaCaT) and UVB to establish a model, after the compound to be test was applied to HaCaT, the expression levels of *TNF-α, IL-8, IL-1β* and *EP2* were detected; using immortalized keratinocytes (HaCaT) and capsaicin to establish a model, after the compound to be test was applied to HaCaT, the expression level of *TRPV1* was detected, so as to evaluate the soothing effect of the test substance from multiple dimensions.

### Test Example 6-1: Detection of TNF-α, IL-8, IL-1β and EP2 gene expression in HaCaT after UVB radiation

1) Cell inoculation: HaCaT cells were inoculated into a 6-well plate at 6×10⁵/well, cultured in an incubator (37°C, 5% CO₂) for 12 hrs, and a normal control group (0 mJ/cm² + vehicle control), a model control group (80 mJ/cm² + vehicle control), a low concentration group (80 mJ/cm² + low concentration compound), a medium concentration group (80 mJ/cm² + medium concentration compound), and a high concentration group (80 mJ/cm² + high concentration compounds) were respectively provided.
2) UVB modeling: The HaCaT cells were washed 3 times with D'Hanks before UVB radiation, 1 mL of D'Hanks was added into the wells, to make the cells to be immersed, and the control group was coated with tinfoil and placed in the dark. For the test groups, UVB modeling (80 mJ/cm²) was performed on the group requiring radiation, respectively.
3) Administration: DMEM medium containing different concentrations of compound were added to each test group for continued culture for 24 hrs.
4) After the culture was completed, cell sampling was performed, total RNA of each test group was extracted, to synthesize cDNA, and gene expression level of *β-actin* and target gene was detected by q-PCR.
5) The relative expression level of RNA of the target gene was calculated by using β-actin as internal reference of gene expression. $RNA Relative Espression level = {2}^{ΔΔC \left(t\right)}$ $ΔΔC \left(t\right) = \overline{ΔC {\left(t\right)}_{model control group}} - ΔC {\left(t\right)}_{sample group}$ $ΔC \left(t\right) = C {\left(t\right)}_{target gene} - C {\left(t\right)}_{β - actin}$

### Test Example 6-2: Detection of TRPV1 gene expression in HaCaT after capsaicin stimulation

1) Cell inoculation: HaCaT cells were inoculated into a 6-well plate at 6 × 10⁵/well, cultured in an incubator (37 °C, 5% CO₂) for 12 hrs, and a normal control group (0 µM capsaicin + vehicle control), a model control group (0 µM capsaicin + vehicle control), a low concentration group (15 µM capsaicin + low concentration compound), a medium concentration group (15 µM capsaicin + medium concentration compound), and a high concentration group (15 µM capsaicin + high concentration compound) were respectively provided.
2) Induction and administration: The culture solution in the 6-well plate was discarded and the administration operation was carried out. The culture solution containing the test substance and the capsaicin stock solution was added according to the above drug group, and 1 mL was added to each well. After the administration, the 24-well plate was placed in the incubator (37 °C, 5% CO₂) for 24 hrs ± 2 hrs.
3) After incubation, D-Hanks was used to gently rinse cells once or twice, the normal control group was added with fresh medium, the sample group was added with fresh medium containing the corresponding concentration of the compound, and the cells were cultured at 37° C and 5% CO₂ for 24 hrs.
4) After the culture was completed, cell sampling was performed, total RNA of each test group was extracted to synthesize cDNA, and gene expression of *β-actin* and target gene was detected by q-PCR.
5) The relative expression level of RNA of the target gene was calculated by using *β-actin* as internal reference of gene expression. $RNA Relative Espression level = {2}^{ΔΔC \left(t\right)}$ $ΔΔC \left(t\right) = \overline{ΔC {\left(t\right)}_{model control group}} - ΔC {\left(t\right)}_{sample group}$ $ΔC \left(t\right) = C {\left(t\right)}_{target gene} - C {\left(t\right)}_{β - actin}$

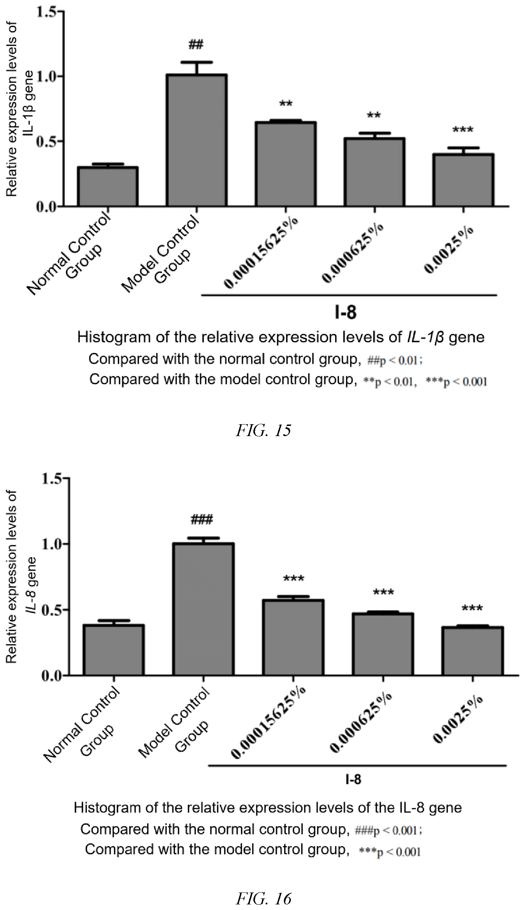

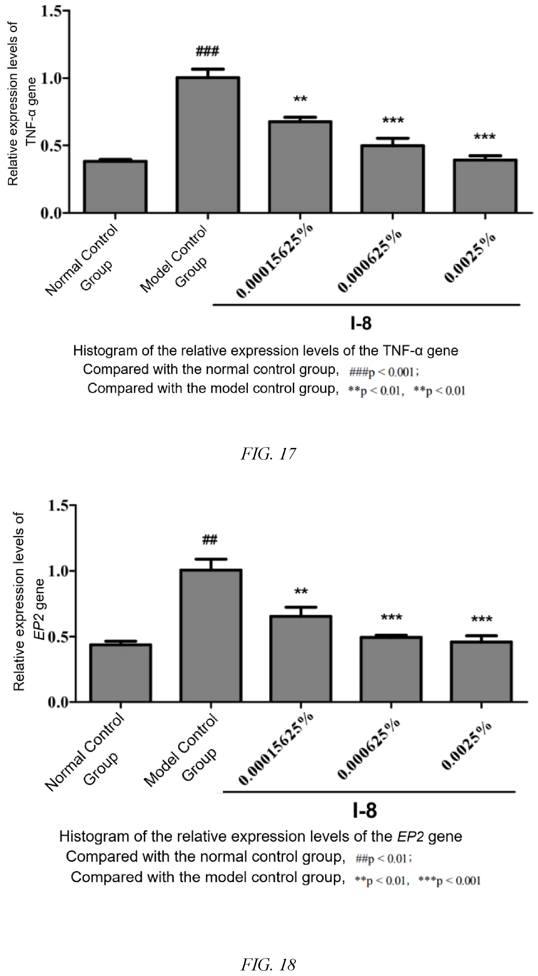

As shown in FIGS. 15-19, compound I-8 is capable of downregulating the expression level of *TNF-α, IL-8, IL-1β, EP2* and *TRPV1* in a dose-dependent manner, indicating that compound I-8 has a soothing effect.

### Test Example 7: Evaluation of the antioxidant efficacy of the compound using HaCaT human immortalized keratinocytes

Oxidative reactions such as respiration and metabolism in organisms generate reactive free radicals. Under normal bodily conditions, free radicals have a stable scavenging system, so that the free radical content of the body is kept at a relatively low concentration. Oxidative stress (OS) refers to an imbalance between oxidation and antioxidation in the body due to endogenous and/or exogenous stimuli, resulting in excessive free radical production. Excessive free radicals produce a series of negative effects in the body, including oxidative damage to biomolecules and further causing cell death and tissue damage, etc. In organisms, almost all ROS, except for trace amounts utilized by the body, should be promptly scavenged. Medium Wave Erythema Effect Ultraviolet (UVB) (280-319 nm) radiation damage is the most important factor leading to photoaging of the skin, primarily damaging keratinocytes (HaCaT), specifically manifested as the accumulation of photoproducts, increased ROS, and exacerbated oxidative damage, etc. Over long periods of evolution, organisms have developed a complete antioxidant system, maintaining a dynamic balance between the production and elimination of free radicals. This is an adaptive mechanism of organisms, and the main members include antioxidant enzymes, antioxidants, and proteins that separate transition metals, all of which can specifically limit oxidative damage to the body. Therefore, the antioxidant efficacy of a test compound can be evaluated by detecting the ROS scavenging rate in keratinocytes after UVB radiation. Example 7 below uses HaCaT immortalized human keratinocytes to evaluate the antioxidant efficacy of a compound.

### Test Example 7: Detection of ROS content in HaCaT after UVB radiation

1) Cell inoculation: HaCaT cells were inoculated into a 96-well plate at 2 × 10⁴/well, cultured in an incubator (37 °C, 5% CO₂) for 12 hrs, and a control group (120 mJ/cm² + vehicle control), a low concentration group (120 mJ/cm² + low concentration compound), a medium concentration group (120 mJ/cm² + medium concentration compound), and a high concentration group (120 mJ/cm² + high concentration compound) were respectively provided.
2) UVB modeling: The HaCaT cells were washed 3 times with D'Hanks before UVB radiation, 50 µL of D'Hanks was added into the wells, to make the cells to be immersed, and the control group was coated with tinfoil and placed in the dark. For the test groups, UVB modeling was performed on the group requiring radiation, respectively.
3) Administration: DMEM medium containing different concentrations of compound were added to each test group for continued culture for 24 hrs.
4) Transferring the ROS fluorescent probe: DCFH-DA was Diluted to a final concentration of 10 µmol/L using PBS at a dilution ratio of 1:1000. The culture medium in all wells except the bare cell wells was discarded. After washing three times with PBS, 200 µL of DCFH-DA working solution was added to each well, then incubated in a CO₂ incubator for 30 min, and after incubation, the cells in each well were washed with PBS for 3 times.
5) Fluorescence analysis. The 96-well plate to be tested was placed on the detection stage of the fluorescence microplate reader, with the incident light wavelength set to 525 nm, the excitation light wavelength set to 488 nm, and the reading was analyzed.

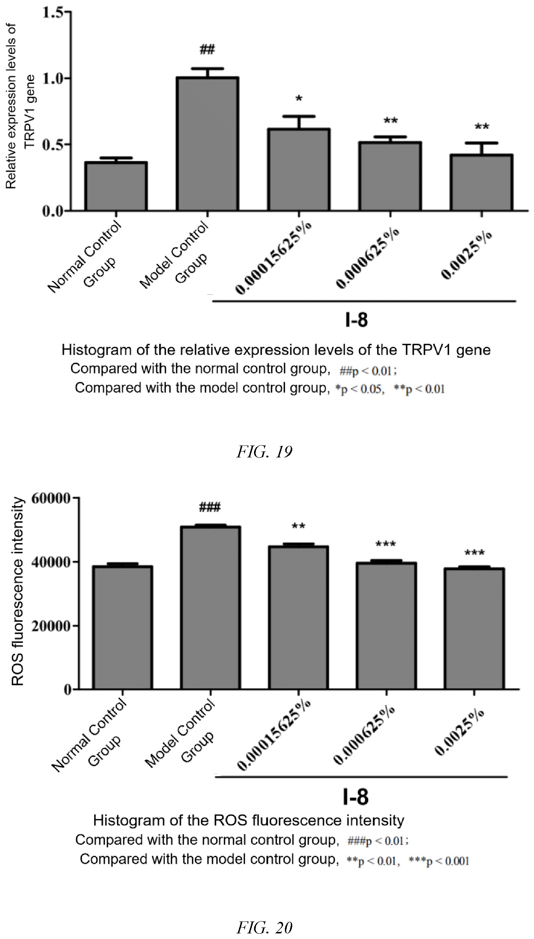

Experimental results: as shown in FIG. 20, compound I-8 is capable of scavenging ROS in a dose-dependent manner. This indicates that compound **I-8** has antioxidant efficacy.

Human skin gradually becomes atrophic (thinner), fragile, exhibits poor pigmentation, and delayed wound healing with age. Skin fragility is partly attributed to changes in hemidesmosomes and the expression downregulation of various collagen proteins (Collagen I, III, IV, VII) or laminins (such as LN-5) at the dermal-epidermal junction. LN-5 has been proven to be a component of anchoring fibers in the basement membrane of the skin, cornea, conjunctiva, and other tissues. LN-5 participates in cell-cell interactions through the mediation of integrins and proteoglycans, thereby playing a crucial role in cell adhesion, growth, migration, and differentiation. The appearance of wrinkles is closely related to the normal synthesis and expression of collagen and laminins. Therefore, the increase of the above collagen content and LN-5 can achieve a certain role in resisting wrinkles, and plays an important role in the aging process of the skin. Therefore, the anti-wrinkle effect of the compound to be tested was evaluated by detecting the level of Collage I protein of epidermal cells (fibroblasts) by ELISA, and detecting the transcription levels of epidermal cells (keratinocytes) III, IV, VII and LN -5 by Real-Time PCR. The following Test Examples 8-1 and 8-2 evaluated the anti-wrinkle efficacy of compounds using HaCaT human immortalized keratinocytes and HSF human skin fibroblasts.

### Test Example 8-1: Detection of Type I Collagen Expression in HSF Human Skin Fibroblasts

1) Cell inoculation: Cells were inoculated into a 96-well plate at 2 × 10⁴/well (37 °C, 5% CO₂), and cultured for 24 hrs.
2) Administration: The culture medium in the 96-well plate was discarded and the administration operation was carried out. The culture medium containing the compound to be test was added into the sample group, and cell culture medium without the compound was added into the control group, 200 µL per well. After the administration, the 96-well plate was placed in an incubator (37 °C, 5% CO₂) and incubated for 24 hrs ± 2 hrs.
3) Detection of Type I collagen: After incubation, cell supernatant was collected and the content of type I collagen was determined by using a human type I collagen enzyme-linked immunosorbent assay kit.

### Test Example 8-2: Detection of Collage III, IV, VII and LN -5 gene expression levels in HaCaT human immortalized keratinocytes

1) Cell inoculation: The epidermal cells were inoculated into a 6-well plate with 1 × 10⁶ cells/well, cultured in an incubator (37 °C, 5% CO₂) for 12 hrs, and a control group (0% compound), a low concentration group (low concentration compound), a medium concentration group (medium concentration compound), and a high concentration group (high concentration compound) were respectively provided.
2) Administration: For the test groups, DMEM medium containing different concentrations and compounds were added to continue to culture for 24 hrs.
3) Cell collection: After culture, cell samples were collected for subsequent *Collage III, IV, VII* and *LN-5* expression detection.

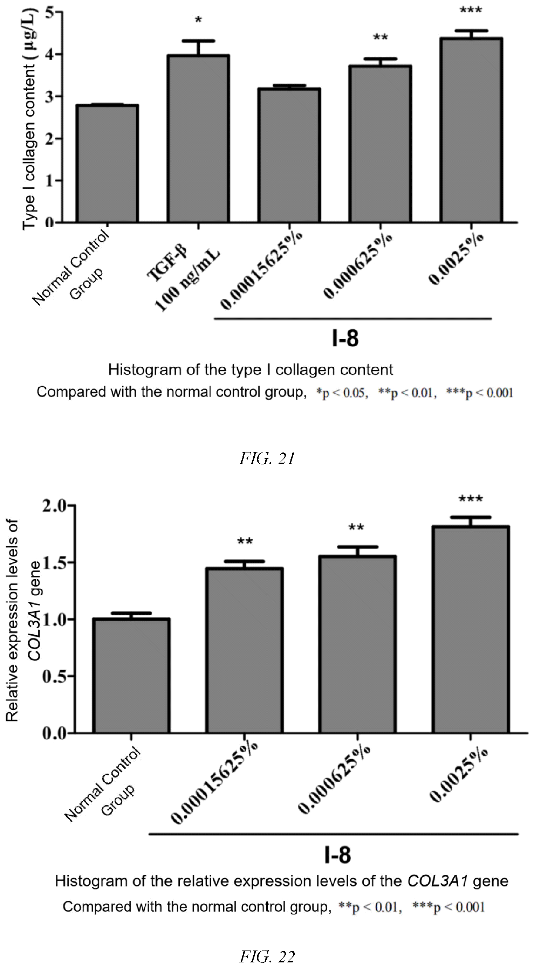

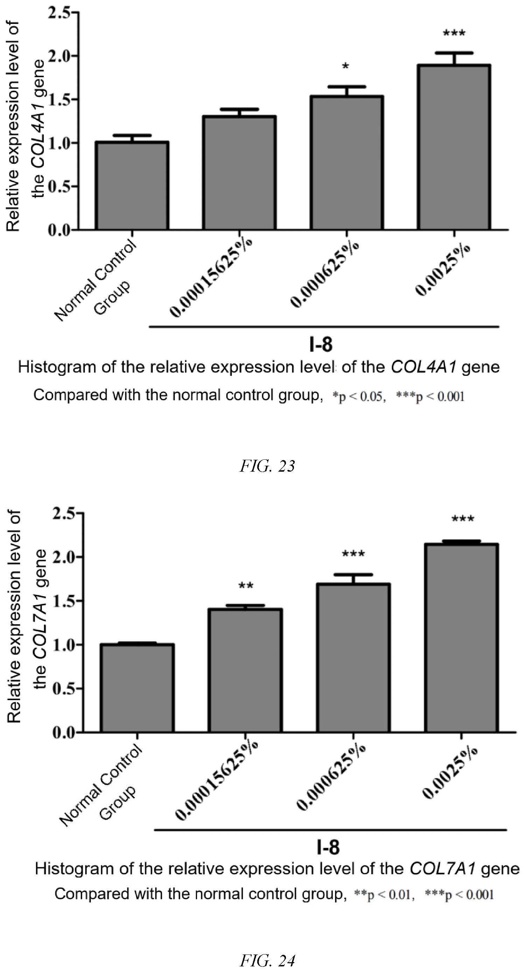

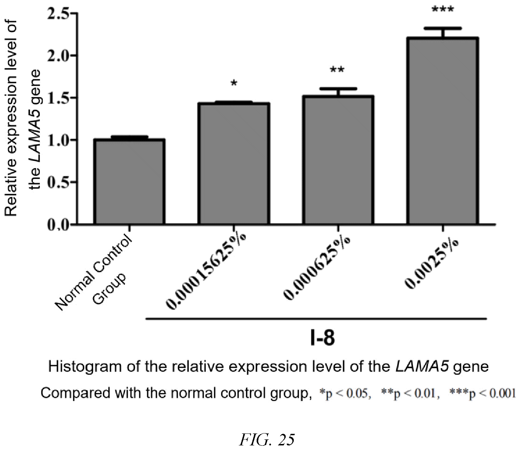

As shown in FIG. 21 to FIG. 25, compound **I-8** is capable of improving the secretion of type I collagen, upregulate *COL341, COL4A1, COL7A1* and *LAMA5* gene expression in a dose-dependent manner. This indicates that compound **I-8** has anti-wrinkle efficacy.

### Test Example 9: Detection and Calculation of ROS scavenging rate of each compound in HaCaT cells after UVB radiation

Using the experimental method in Test Example 7, the ROS scavenging rate of each compound in HaCaT cells after UVB radiation was calculated according to the following formula, and the results are listed in Table 1. $ROS scavenging rate \left(%\right) = \frac{S \left(Model control group\right) - S \left(Sample group\right)}{S \left(Model control group\right)} \times 100 %$ wherein S is fluorescence intensity.

**TABLE 1**

| Compounds (content) | ROS scavenging rate - % | Compounds (content) | ROS scavenging rate -% |
|---|---|---|---|
| I-1 (0.0025%) | 5 | I-14 (0.0025%) | 29 |
| I-2 (0.0025%) | 41 | I-15 (0.0025%) | 27 |
| I-3 (0.0025%) | 6 | I-16 (0.0025%) | 38 |
| I-4 (0.0025%) | 37 | I-17 (0.0025%) | 2 |
| I-5 (0.0025%) | 31 | I-18 (0.0025%) | 21 |
| I-6 (0.0025%) | 28 | I-19 (0.0025%) | 19 |
| I-7 (0.0025%) | 40 | I-20 (0.0025%) | 37 |
| I-8 (0.00015625%) | 12 | I-21 (0.0025%) | 8 |
| I-8 (0.000625%) | 22 | I-22 (0.0025%) | 9 |
| I-8 (0.0025%) | 26 | I-23 (0.0025%) | 34 |
| I-9 (0.0025%) | 11 | I-24 (0.0025%) | 31 |
| I-10 (0.0025%) | 15 | I-25 (0.0025%) | 29 |
| I-11 (0.0025%) | 12 | I-26 (0.0025%) | 36 |
| I-12 (0.0025%) | 33 | I-27 (0.0025%) | 35 |
| I-13 (0.0025%) | 35 | I-28 (0.0025%) | 30 |

Those of ordinary skill in the art can understand that the above embodiments are specific examples for implementing the present invention, and in practical applications, various changes can be made in form and detail without departing from the spirit and scope of the present invention.

## Claims

1. A compound having a structure represented by the following general formula I, or a salt, stereoisomer or solvate thereof, 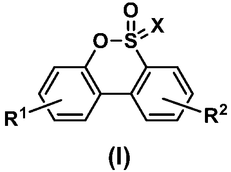 wherein X is oxygen or absent;
R¹ and R² are independently hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, -OC(O)R¹⁻³, -OCH₂OC(O)R¹⁻⁴, halogen, cyano, nitro, -C(O)R¹⁻⁵ or -COOR¹⁻⁶;
R¹⁻¹, R¹⁻², R¹⁻³, R¹⁻⁴, R¹⁻⁵ and R¹⁻⁶ are independently hydrogen, C₁₋₆ alkyl, halogen-substituted C₁₋₆ alkyl or -NR^{a}R^{b};
R^{a} and R^{b} are independently hydrogen, C₁₋₆ alkyl or halogen-substituted C₁₋₆ alkyl.

2. The compound, or salt, stereoisomer or solvate thereof according to claim 1, **characterized in that** X is oxygen.

3. The compound, or salt, stereoisomer or solvate thereof according to claim 1,
**characterized in that** the C₁₋₆ alkyl is methyl, ethyl, n-propyl, isopropyl, n-butyl, isobutyl or tert-butyl;
and/or, the C₁₋₆ alkoxy is methoxy, ethoxy, n-propoxy, isopropoxy, n-butoxy, isobutoxy or tert-butoxy;
and/or, the halogen is fluorine, chlorine, bromine or iodine;
and/or, the halogen-substituted C₁₋₆ alkyl is a halogen-substituted methyl, a halogen-substituted ethyl, a halogen-substituted n-propyl, a halogen-substituted isopropyl, a halogen-substituted n-butyl, a halogen-substituted isobutyl or a halogen-substituted tert-butyl.

4. The compound, or salt, stereoisomer or solvate thereof according to claim 1, **characterized in that** R¹ and R² are independently hydrogen, hydroxy, C₁₋₆ alkyl, C₁₋₆ alkoxy, amino, -N-C(O)R¹⁻¹, -NR^{a}R^{b}, -CH₂C(O)OR¹⁻², phenyl, -OC(O)R¹⁻³ or - OCH₂OC(O)R¹⁻⁴.

5. The compound, or salt, stereoisomer or solvate thereof according to claim 1, **characterized in that** R¹ and R² are independently hydrogen, hydroxy, C₁₋₆ alkoxy, 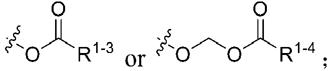 wherein, R¹⁻³ and R¹⁻⁴ are independently methyl, ethyl, n-propyl, isopropyl, n-butyl, isobutyl, tert-butyl or

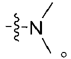

6. The compound, or salt, stereoisomer or solvate thereof according to claim 1, **characterized in that** R¹ and R² are independently hydroxy, methoxy, ethoxy, n-propoxy, isopropoxy, n-butoxy, isobutoxy, tert-butoxy,

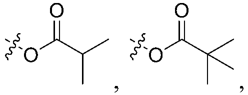

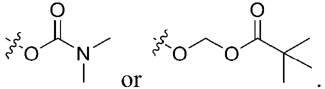

7. The compound, or salt, stereoisomer or solvate thereof according to any one of claims 1 to 6, **characterized in that** the compound is selected from any one of the following:
| Name | Structural Formula |
|---|---|
| I-1 | 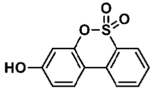 |
| I-2 | 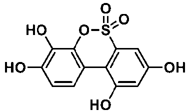 |
| I-3 | 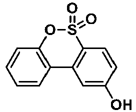 |
| I-4 | 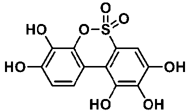 |
| I-5 | 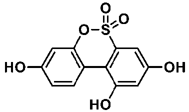 |
| I-6 | 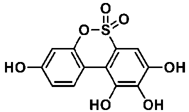 |
| I-7 | 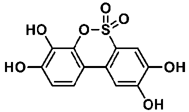 |
| I-8 | 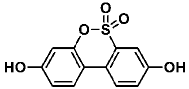 |
| I-9 | 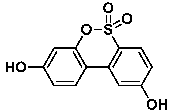 |
| I-10 | 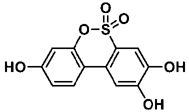 |
| I-11 | 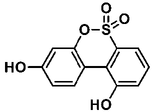 |
| I-12 | 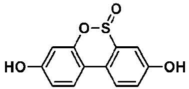 |
| I-13 | 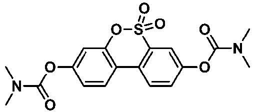 |
| I-14 | 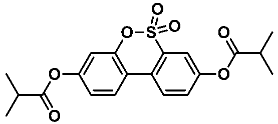 |
| I-15 | 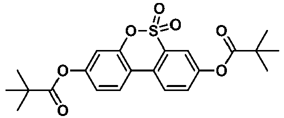 |
| I-16 | 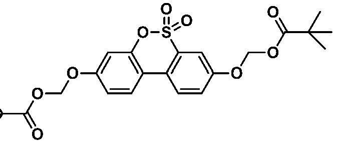 |
| I-17 | 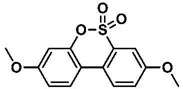 |
| I-18 | 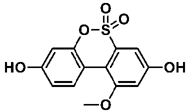 |
| I-19 | 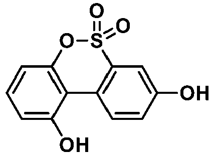 |
| I-20 | 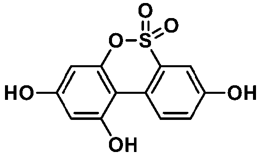 |
| I-21 | 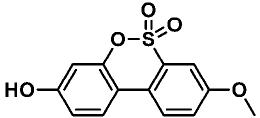 |
| I-22 | 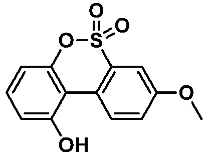 |
| I-23 | 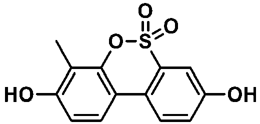 |
| I-24 | 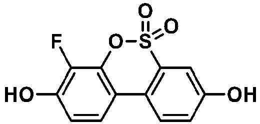 |
| I-25 | 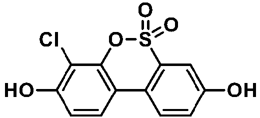 |
| I-26 | 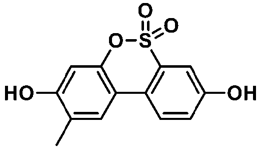 |
| I-27 | 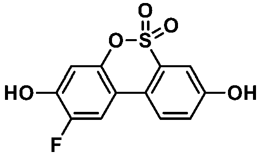 |
| I-28 | 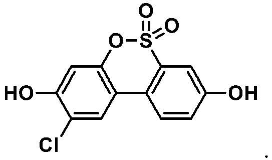 |

8. A composition comprising the compound, or salt, stereoisomer or solvate thereof according to any one of claims 1 to 7; and a cosmetically acceptable excipient.

9. Use of the compound, or salt, stereoisomer or solvate thereof according to any one of claims 1 to 7, or the composition according to claim 8 in the preparation of cosmetics.

10. A method for alleviating skin inflammation and/or reducing reactive oxygen species in skin cells, **characterized in that** the method comprises the steps of:
applying the compound, or salt, stereoisomer or solvate thereof according to any one of claims 1 to 7 to a subject; or applying the composition according to claim 8 to a subject.
